# EUROPEAN PATENT APPLICATION

(11) **EP 2 430 927 A1**
(43) Date of publication of application: **21.03.2012**
(21) Application number: 10172304.7
(22) Date of filing: 09.08.2010
(51) Int. Cl.: A23L 1/30, A61K 31/575, A61P 1/16

(54) **Method for treating fatty liver diseases, in particular non-alcoholic steatohepatitis**

(71) Applicant: Universiteit Maastricht, 6211 LK Maastricht (NL); Academisch Ziekenhuis Maastricht, 6229 HX Maastricht (NL)
(72) Inventor: SVERDLOV, Ronit, 6228 SB, MAASTRICHT (NL); BIEGHS, Veerle, 3650, DILSEN (BE); GORP, VAN, Patrick Johannes Jacobus, 6301 LR, VALKENBURG a/d GEUL (NL)
(74) Representative: Habets, Winand

(57) **Abstract**

The invention is in the field of medical treatments of liver disease, in particular non-alcoholic fatty liver diseases, such as non-alcoholic steatohepatitis. The invention provides a composition comprising an effective amount of a compound capable of increasing the intracellular level of 27-hydroxycholesterol for the treatment of liver inflammation in fatty liver disease. It also provides a method of reducing liver inflammation in a patient with non-alcoholic fatty liver disease by administering an effective amount of 27-hydroxycholesterol.

## Description

### Field of the invention

The invention is in the field of prevention and medical treatment of liver diseases, in particular fatty liver disease, more in particular non-alcoholic fatty liver disease, such as non-alcoholic steatohepatitis.

### Background of the invention

Non alcoholic fatty liver disease (NAFLD) is a condition ranging from benign lipid accumulation in the liver (steatosis) to steatosis combined with inflammation. The latter is referred to as non-alcoholic steatohepatitis (NASH). NASH is viewed as the hepatic event of the metabolic syndrome. Estimates in USA are that 5.7-17% of all adults have NASH, while 17% to 33% of Americans have NAFLD. NASH and NAFLD are frequently reported in both men and women, although it most often appears in women and is especially prevalent in the obese. As obesity and insulin resistance reach epidemic proportions in industrialized countries, the prevalence of both NAFLD and NASH is increasing and is therefore considered as a major health hazard.

Steatosis alone is considered a relatively benign and reversible condition. However, the transition towards NASH represents a key step in the pathogenesis, as it will set the stage for further damage to the liver, such as fibrosis, cirrhosis and liver cancer.

Currently, liver biopsy is used in the clinical practice as the primary method for detection of liver inflammation in NASH.

There is no established therapy for patients suffering from NASH. Therapy is, therefore, focused mainly on risk factors, weight reduction and pharmacological intervention. Promising pharmacological treatments have been demonstrated with antioxidants, insulin sensitizers, hepatoprotectants and lipid-lowering agents.

### Summary of the invention

The present invention provides methods and compositions for the prevention or treatment of non-alcoholic fatty liver disease (NAFLD) and conditions associated with NAFLD. More in particular, it provides a method for the prevention or treatment of non-alcoholic steatohepatitis, (NASH).

The disclosure provides that such diseases may be prevented or treated by administering a therapeutically effective amount of a composition comprising a compound capable of increasing the intracellular level of 27-hydroxycholesterol (27-HC).

In other terms, the invention relates to a pharmaceutical composition comprising a compound capable of increasing the intracellular level of 27 hydroxycholesterol for use in the treatment of non-alcoholic fatty liver disease.

This invention is also directed to a combination therapy treatment for treating Metabolic Syndrome. The Metabolic Syndrome is a set of metabolic abnormalities including centrally distributed obesity, hyperlipideniia, elevated triglycerides, elevated blood pressure, cardiovascular diseases, Type II Diabetes and NAFLD. As part of a combination therapy regime for the treatment of Metabolic Syndrome, pharmaceutical formulations of 27-HC are used for treating the NAFLD (and NASH), component of Metabolic Syndrome.

### Legend to the figures

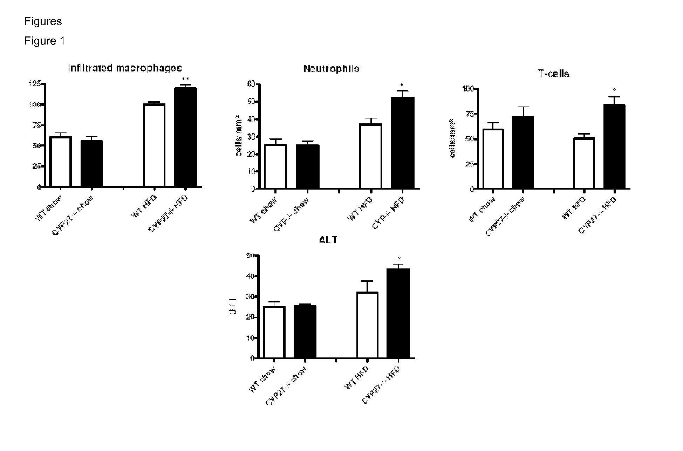

Figure 1: Graphs showing that hepatic inflammation and liver damage is increased in mice transplanted with bone marrow from Cyp27A1 knock out mice as indicated by the increased number of inflammatory cells and ALT levels.

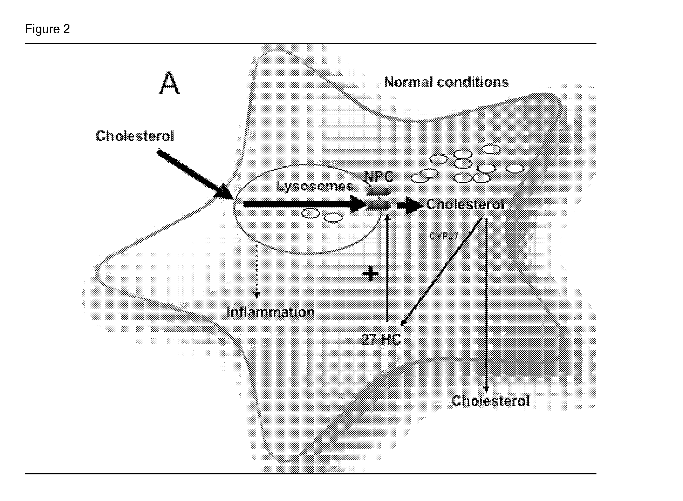

Figure 2: Model showing the role of 27-HC in reducing the levels of lysosomal cholesterol levels.

### Detailed description of the invention

Currently, the mechanisms that drive hepatic inflammation during the progression of NASH are largely unknown. We found that lysosomal fat accumulation in Kupffer cells is the underlying mechanism of inflammation in non-alcoholic fatty liver disease, in particular in non-alcoholic steatohepatitis (NASH).

Our study shows for the first time that liver inflammation is induced when 27-HC levels are decreased. We were also able to show that increased levels of 27-HC remedied the problem of lysosomal cholesterol accumulation and that liver inflammation could be ameliorated or cured by increasing 27-HC levels in vivo.

Hence, the invention relates to a method of reducing liver inflammation in a patient with fatty liver disease by administering an effective amount of a compound capable of increasing the intracellular level of 27-HC.

One way of increasing the intracellular level of 27-HC is to administer a compound comprising 27-HC as an active component. 27-HC may be contained in a pharmaceutically acceptable carrier, and may be administered either orally, parenterally, by injection, intravenous infusion, inhalation, controlled dosage release or by intraperitoneal administration for the treatment or alleviation of NAFLD. The preferred method of administration is orally.

Endogenously produced 27-HC may also be used in a method according to the invention. Generally, such a method involves providing a compound increasing the intracellular level of 27-HC by increasing the in vivo production of 27-HC. Such a compound may be sterol 27-hydroxylase CYP27A1.

Enhancement of naturally occurring 27-HC may also be accomplished by in vivo administration into cells competent for the production of 27-HC, a vector comprising and expressing a DNA sequence encoding biologically active sterol 27-hydroxylase CYP27A1. Inside KCs, the breakdown of free cholesterol by Cyp27A1 results in the formation of 27-hydroxycholesterol. The vector used may be a viral vector, including but not limited to a lentivirus, adenovirus, adeno-associated virus and virus-like vectors, a plasmid, or a lipid vesicle. The vector is taken up by the cells competent for the production of biologically active 27-HC.The DNA sequence is expressed and the biologically active 27-HC is produced.

In one embodiment, the fatty liver disease is non-alcoholic fatty acid liver disease (NAFLD), more in particular non-alcoholic steatohepatitis (NASH

The method according to the invention may be used for the treatment of the diseases mentioned herein but may also be used prophylactic, i.e. in order to prevent the diseases from occurring or ameliorating the inflammatory effects once the disease occurs.

27-HC may be administered via the diet or as a drug or a pro-drug, orally, intravenously or in any other suitable way. Alternatively, the in vivo bioavailability of 27-HC may be increased by interfering with the in vivo pathway that produces 27-HC. For example, the bioavailability of 27-HC may be increased by increasing the activity of CYP 27 or increasing the expression level of CYP 27A1.

If administered as a drug or a food supplement, the 27-HC is to be administered in an effective dose or an effective amount. The phrase "effective dose" or "effective amount" refers to that dose or amount of a substance that produces some desired local or systemic effect at a reasonable benefit/risk ratio applicable to any treatment. The effective amount of such substance will vary depending upon the subject and disease condition being treated, the weight and age of the subject, the severity of the disease condition, the manner of administration and the like, which can readily be determined by one of ordinary skill in the art.

More in particular, the total daily dose of 27-HC is usually in the milligram range per kilogram body weight, such as between 5 and 100 milligram per kilogram body weight, more in particular about 40 milligram per kilogram body weight. In yet another embodiment, the 27-HC composition is administered at a frequency of 4 or less times per day (e.g., one, two or three times per day).

The terms "therapeutic agent," and "active agent" may be used interchangeably and refer to a substance, such as a chemical compound or complex, that has a measurable beneficial physiological effect on the body, such as a therapeutic effect in treatment of a disease or disorder, when administered in an effective amount. Further, when these terms are used, or when a particular active agent is specifically identified by name or category, it is understood that such recitation is intended to include the active agent per se, as well as pharmaceutically acceptable, pharmacologically active derivatives thereof, or compounds significantly related thereto, including without limitation, salts, pharmaceutically acceptable salts, N-oxides, prodrugs, active metabolites, isomers, fragments, analogs, solvates hydrates, radioisotopes, etc.

The term "Metabolic Syndrome" is used to define a set of conditions that places people at high risk for coronary artery disease. These conditions include Type II diabetes, central obesity also known as visceral adiposity, high blood pressure, and a poor lipid profile with elevated LDL ("bad") cholesterol, low HDL ("good") cholesterol, elevated triglycerides. All of these conditions are associated with high blood insulin levels. Associated diseases include NAFLD (especially in concurrent obesity) polycystic ovarian syndrome; hemochromatosis (iron overload); acanthosis nigricans (a skin condition featuring dark patches); Non-alcoholic steatohepatitis (NASH - an extreme form of fatty liver) .

The term "NAFLD" (non-alcohol fatty liver disease) refers to a group of conditions where there is an accumulation of excess fat in the liver of people who drink little or no alcohol. In fatty liver disease, fat accumulates in the liver cells. A subgroup of people with NAFLD may have a more serious condition termed non-alcoholic steatohepatitis (NASH). In NASH, fat accumulation is associated with liver cell inflammation and different degrees of scarring. Cirrhosis occurs when the liver sustains substantial damage, and the liver cells are gradually replaced by scar tissue which results in the inability of the liver to work properly. The use of the term "NAFLD" is used to include all conditions reflecting a form of non-alcohol fatty liver disease, but in particular, NASH.

The term "pharmaceutically acceptable salts" is art-recognized and refers to the relatively non-toxic, inorganic and organic acid addition salts of compounds, including, for example, those contained in compositions of the present invention.

The term "pharmaceutically acceptable carrier" is art-recognized and refers to a pharmaceutically-acceptable material, composition or vehicle, such as a liquid or solid filler, diluent, excipient, solvent or encapsulating material, involved in carrying or transporting any subject composition or component thereof from one organ, or portion of the body, to another organ, or portion of the body. Each carrier must be acceptable in the sense of being compatible with the subject composition and its components and not injurious to the patient. Some examples of materials which may serve as pharmaceutically acceptable excipients include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) IV fluids, including but not limited to Ringer's solution, 5% dextrose in water, and half normal saline; (19) ethyl alcohol; (20) phosphate buffer solutions; and (21) other non-toxic compatible substances employed in pharmaceutical formulations. [0032] The term "Pharmacological Chaperones "refers to small molecules which stabilize the correct folding of a protein and are administered to the patient resulting in a recovery of function lost due to mutation.

The term "prophylactic" or "therapeutic" treatment is art-recognized and refers to administration to the host of one or more of the subject compositions. If it is administered prior to clinical manifestation of the unwanted condition (e.g., disease or other unwanted state of the host animal) then the treatment is prophylactic, i.e., it protects the host against developing the unwanted condition, whereas if administered after manifestation of the unwanted condition, the treatment is therapeutic (i.e., it is intended to diminish, ameliorate or maintain the existing unwanted condition or side effects therefrom).

The term "treating" is art-recognized and refers to curing as well as ameliorating at least one symptom of any condition or disease.

NAFLD and NASH in particular, involve the development of histologic changes in the liver that are comparable to those induced by excessive alcohol intake but in the absence of alcohol abuse. Macrovesicular and/or microvesicular steatosis, lobular and portal inflammation, and occasionally Mallory bodies with fibrosis and cirrhosis characterize NAFLD. NAFLD (and NASH in particular) is also commonly associated with hyperlipidemia, obesity, hypertension, atherosclerosis, and Type II diabetes mellitus, commonly known as The Metabolic Syndrome. Other clinical conditions characterized by hepatic steatosis and inflammation include excessive fasting, jejunoileal bypass, total parental nutrition, chronic hepatitis C, Wilson's disease, and adverse drug effects such as those from corticosteroids, calcium channel blockers, high dose synthetic estrogens, methotrexate and amiodarone. Thus, the term "non-alcoholic steatohepatitis" can be used to describe those patients who exhibit these biopsy findings, coupled with the absence of (a) significant alcohol consumption, (b) previous surgery for weight loss, (c) history of drug use associated with steatohepatitis, (d) evidence of genetic liver disease or (e) chronic hepatitis C infection. See, J. R. Ludwig et al., Mayo Clin. Proc, 55, 434 (1980) and E. E. Powell et al., Hepatol., 11, 74 (1990).

Hepatic steatosis (fatty liver) and steatohepatitis (fatty liver with inflammation or scarring) are two forms of the fatty liver disease that develops in children and adults, frequently associated with being overweight or obese. This type of liver disease is often referred to as non-alcoholic steatohepatitis (NASH), because it occurs in the absence of a significant amount of alcohol intake.

### Example 1: Link between uptake of modified lipids and hepatic inflammation.

To evaluate the involvement of uptake of modified lipoproteins by Kupffer cells (Cs) in the development of diet-induced NASH, female low-density lipoprotein receptor-deficient (Ldlr) knock-out mice were lethally irradiated and transplanted with bone marrow from Msr1/Cd36 double knock-out mice or WT mice and fed a Western diet. Macrophage and neutrophil infiltration revealed that hepatic inflammation was substantially reduced by approximately 30% in Msr1/Cd36 double knock out transplanted mice compared with control mice. Consistent with this, the expression levels of well-known inflammatory mediators were reduced.

Apoptotis and fibrosis were less pronounced in Msr1/Cd36 double knock out transplanted mice, in addition to the protective phenotype of natural antibodies against oxidized low-density lipoprotein in the plasma. Surprisingly, the effect on hepatic inflammation was independent of foam cell formation.

It was concluded that it is not the total amount of lipids in Kupffer cells that will trigger inflammation, but rather the type of lipids or the mechanism of internalization.

### Example 2: Link between hepatic inflammation in NASH and the intracellular fat localization in Kupffer cells.

Ldlr knock out mice were depleted from their hematopoietic system by irradiation and were transplanted with bone marrow from mice lacking either Sr-a or Cd36. Control mice were injected with wild-type (Wt) bone marrow. After a recovery period of 9 weeks, the mice were put on a high fat diet (HFD) for 3 months.

It was found that Cd36 knock out and Sr-a⁻ knock out transplanted (tp) mice showed reduced inflammatory markers in blood and liver compared to Wt-tp mice. Moreover, fibrosis was also less pronounced in both Cd36 knock out and Sr-a⁻ knock out tp mice compared to Wt-tp mice. In plasma, the protective IgM auto-antibodies were increased in both Cd36 knock out and Sr-a knock out -tp mice compared to Wt-tp mice. Despite the lack of difference in the appearance of foamy Kupffer cells (KC's), electron microscopy (EM) revealed a significant difference in lysosomal/cytoplasmic cholesterol storage. It was observed that the Wt-tp mice showed increased lysosomal fat whereas the Cd36 knock out - and Sr-a⁻ knock out tp mice showed more cytoplasmic fat inside the KC's.

It was concluded that the reduction in inflammation is correlated with decreased lysosomal cholesterol accumulation in Kupffer cells. These observations establish the link between lysosomal fat accumulation in KC's and hepatic inflammation.

### Example 3: expression of CYP27A1 in Kupffer cells can modulate lysosomal fat accumulation in vitro

We incubated bone marrow derived macrophages from Cyp27A1 knock-out mice and Wt mice with ox LDL for 24 hours. It was found that cholesterol levels in lysosomes were increased. Consequently, cholesterol was significantly more crystallized in macrophages of the Cyp27A1 knock out mice compared to Wt mice.

Since the breakdown of free cholesterol by Cyp27A1 results in the formation of 27-hydroxycholesterol it may be concluded that 27-HC can reduce lysosomal cholesterol accumulation in macrophages.

### Example 4: expression of CYP27A1 in Kupffer cells can modulate lysosomal fat accumulation and thereby hepatic inflammation.

Ldlr knock-out mice were transplanted with bone marrow from mice lacking *Cyp27A1.* Control mice were transplanted with wild-type (Wt) bone marrow. After a recovery period of 9 weeks, the mice were put on chow or high fat high cholesterol (HFC) diet for 3 months.

It was found that Kupffer cells of the *Cyp27A1*^{*-*/}*⁻-tp* mice showed increased lysosomal fat accumulation. Additionally, cholesterol was more crystallized in *Cyp27A1*^{*-*/*-*}-tp mice compared to *Wt*-tp mice. Surprisingly, CD68 staining revealed that KC's were less foamy upon HFC feeding in *Cyp27A1*^{*-*/*-*}-tp mice. Liver histology demonstrated that *Cyp27A1*^{*-*/*-*}-tp mice had increased hepatic inflammation compared to Wt-tp mice as indicated by the elevated numbers of infiltrated macrophages, neutrophils and T-cells. These findings were confirmed by hepatic expression of *Tnf, II-1b* and *II*-6.

It was concluded that CYP27A1 plays an important role in liver inflammation in NASH, in particular in diet-induced NASH.

It may therefore be concluded that there is a clear association between lysosomal fat accumulation and hepatic inflammation and that inflammation may be decreased by increasing the level or activity of 27-HC.

### Example 5: Liver inflammation may be ameliorated or treated by administration of 27-HC to a mammal

To determine whether exogenous 27-HC can reduce hepatic inflammation, hyperlipidemic LDLR knock-out mice may be fed either chow, HFC diet or HFC diet supplemented with 27-hydroxycholesterol (0.1%w/w) for 3 weeks. Then, the mice may be sacrificed and detailed characterization of liver and plasma may be performed.Mice receiving diet supplemented with 27-HC will show less lysosomal cholesterol accumulation and reduced liver and systemic inflammation, compared to mice receiving HFC diet.

### Example 6: Materials and methods used in the other examples

### Mice

Female *Ldlr*^{*-*/*-*} mice were lethally irradiated and transplanted with *Wt, Cd36*^{*-*/*-*} and *Msr*^{*-*/-}bone marrow. After a recovery period of 9 weeks, the mice were given a HFC diet for 3 months (n=9 in Wt-tp mice, n=8 in both Cd36-/--tp and Msr1-/--tp mice).

Collection of blood, specimens, biochemical determination of lipids in plasma and liver, liver histology, RNA isolation, cDNA synthesis and qPCR, determination of chimerism, aminotransferases, oxysterols and auto-antibody titers against modified LDL are described previously (Bieghs, V., et al.,. Gastroenterology, 2010).

### Blood leukocyte analysis

Sixty microliter of total blood was washed extensively in erythrocyte lysis buffer (0.155 M NH₄CL, 10 mM NaHCO₃ for leukocyte analysis. Cells were stained for monocytes (Mac1-PE), granulocytes (Gr1-FITC), B-cells (6B2-PE) and T-cells (KT3-FITC) (BD Sciences Pharmingen, San Diego, California, USA) in PBS containing 5% normal mouse serum and 1% FCS. After 1 hour of incubation, cells were washed and analyzed by FACS analysis (Facssort, BD Sciences Pharmingen).

### Electron microscopy

Detailed overview about (post)fixation, embedding, cutting and type of electron microscope is described previously (Hepatology). Scoring of the electron microscopy pictures is performed by a trained specialist. Fifty Kupffer cells per mouse were analyzed and scored. The Kupffer cells were divided into five different categories, e.g. primary lysosomes, secondary lysosomes, fatty lysosomes, cytoplasmic fat accumulation and degenerated lysosomes and the percentage of each subtype is calculated. Furthermore, the secondary lysosomes were scored from 0 to 3 for their fat content and complexicity, where score 0 indicates not positive and score 3 indicates extreme fat accumulation inside the lysosomes of the KCs / extremely complex indicated by electrodense particles. The same scoring index is used for the cytoplasmic fat accumulation inside Kupffer cells.

### Statistical analysis

Data was statistically analyzed by performing one-way ANOVA test with a Bonferroni post-test using GraphPad Prism for comparing Wt-tp, *Cd36*^{*-*/*-*}-tp and *Msf*^{*-*/*-*}-tp mice with each other. Data were expressed as the mean ± SEM and considered significant at p<0.05. *, ** and *** indicate p < 0.05, 0.01 and 0.001 resp.

## Claims

1. Composition comprising an effective amount of a compound capable of increasing the intracellular level of 27 hydroxycholesterol or a pharmaceutically acceptable salt thereof for use in the prevention or treatment of liver inflammation in non-alcoholic fatty liver disease.

2. Composition for use according to claim 1 wherein the non-alcoholic fatty liver disease is non-alcoholic steatohepatitis.

3. Composition for use according to claims 1 or 2 wherein the compound capable of increasing the intracellular level of 27 hydroxycholesterol is 27 hydroxycholesterol.

4. Composition for use according to claims 1 - 3 wherein the composition comprises a pharmaceutically acceptable carrier.

5. Composition for use according to claims 1-4 wherein the composition is a food composition or a food supplement.

6. Method for reducing or preventing liver inflammation in a subject in need of such a treatment by administering a compound capable of increasing the intracellular level of 27-hydroxycholesterol or a pharmaceutically acceptable salt thereof.

7. Method according to claim 6 wherein the subject is a patient with non-alcoholic fatty liver disease or a person at risk of developing non-alcoholic fatty liver disease.

8. Method according to claims 6 or 7 wherein the compound capable of increasing the intracellular level of 27 hydroxycholesterol is 27 hydroxycholesterol.

9. Method according to claims 6- 8 wherein the non-alcoholic fatty liver disease is non-alcoholic steatohepatitis.

10. Method according to claims 6-9 wherein the compound also comprises a pharmaceutically acceptable carrier.

11. Method according to claims 6-10 wherein the compound is a food composition or a food supplement.
